# EUROPEAN PATENT APPLICATION

(11) **EP 4 686 942 A1**
(43) Date of publication of application: **04.02.2026**
(21) Application number: 24192599.9
(22) Date of filing: 02.08.2024
(51) Int. Cl.: G01N 33/24, G06Q 50/02, G01N 33/00

(54) **METHOD FOR PROVIDING A VALIDATED MEASURE FOR THE AMOUNT OF CARBON DIOXIDE REMOVED FROM THE ATMOSPHERE BY A REACTANT DEPLOYED ON A FIELD**

(71) Applicant: Inplanet GmbH, 80939 München (DE)
(72) Inventor: Clarkson, Matthew, 8610 Uster (CH); Larkin, Christina, Winchester, SO22 4PX (GB); Swoboda, Philipp, 1180 Wien (AT); Ferrarezi, Jessica, 13416-389 Piracicaba (BR); Maniero Rodrigues, Mayra, 13416-404 Piracicaba (BR); Chiapini, Mariane, 13420-264 Piracicaba (BR); Kluger, Niklas, 13418-220 Piracicaba (BR); Harteneck, Felix, 80939 München (DE)
(74) Representative: Lindner Blaumeier Patent- und Rechtsanwälte

(57) **Abstract**

Method for providing a validated measure (1) for the amount of carbon dioxide removed from the atmosphere by a reactant (2) deployed on a field (3), comprising the steps of: setting up at least one reference experiment (4-7) of a respective experiment type (8-11); analyzing a respective field sample taken from at least one field sampling location (12) in the field (3) and/or performing at least one measurement in-situ at the respective field sampling location (12) to determine a field test result (13) that depends on the amount of carbon dioxide removed from the atmosphere by the reactant (2) at the at least one field sampling location (12); analyzing at least one reference sample taken from the respective reference experiment (4-7) and/or performing at least one measurement in-situ in the respective reference experiment (4-7) to determine a respective reference test result for the respective experiment type (8-11); evaluating a consistency condition (15), wherein a fulfilment of the consistency condition (15) depends on the field test result (13) and the respective reference test result; and providing the validated measure (1) for the amount of carbon dioxide removed from the atmosphere by the reactant (2) deployed on the field (3), when the consistency condition (15) is fulfilled, wherein the validated measure (1) depends on the field test result (13) and/or the respective reference test result.

## Description

The invention concerns a method for providing a validated measure for the amount of carbon dioxide removed from the atmosphere by a reactant deployed on a field.

It is expected, that all pathways that limit global temperature rise to less than 2°C above pre-industrial temperatures will require carbon dioxide removal (CDR) in addition to rapid greenhouse gas emissions reductions. CDR strategies therefore need to rapidly scale over the next few decades in order to reach Paris Agreement Targets.

Terrestrial enhanced weathering is a promising candidate for cost effective and save CDR. This approach involves the acceleration of natural weathering processes via the deployment of crushed rock feedstocks, typically Ca- and Mg-rich silicates, in soils. While models predict this has the potential to remove multiple gigatons of CO₂ annually, the monitoring and verification of carbon removal and storage remains challenging for this approach.

Measurement and monitoring of enhanced weathering necessitates balancing operational and scientific limitations of different approaches. There are different approaches to determine the amount of carbon removed from the atmosphere, primarily based on liquid or solid phase measurements. Solid phase approaches may prove more scalable, since it can e.g. be sufficient to sample the soil once a year, since such approaches provide a time-integrated estimate of CDR. These approaches do however not give direct evidence of bicarbonate formation or export and can overestimate the amount of removed carbon dioxide, e.g. in the presence of different acids then carbonic acid in the soil.

By contrast, liquid phase measurements require more frequent sample collection, but provide direct evidence of bicarbonate formation and export. Moreover, liquid measurements are a source of additional important secondary information, such as a soil water pH-value. Such additional information allows a modelling of dissolved inorganic carbon speciation. Some parameters from liquid measurements, e.g. the amount of dissolved inorganic carbon and the pH-value, can however change due to an outgassing of CO₂, once the liquid sample is extracted, when the liquid is not perfectly sealed within an enclosed space. It can therefore be challenging to use liquid phase measurements in the field.

Various approaches for determining a measure for the amount of carbon dioxide removed from the atmosphere by a reactant in enhanced weathering based on different sets of acquired parameters are, e.g., discussed in the following publications:
Clarkson, M. O. et al. A Review of Measurement for Quantification of Carbon Dioxide Removal by Enhanced Weathering in Soil. (2023) doi: https://doi.org/10.31223/X52D7T;
Reershemius, T. et al. Initial Validation of a Soil-Based Mass-Balance Approach for Empirical Monitoring of Enhanced Rock Weathering Rates. Environ. Sci. Technol. (2023) doi: 10.1021/acs.est.3c03609;
Larkin, C. S. et al. Quantification of CO2 removal in a large-scale enhanced weathering field trial on an oil palm plantation in Sabah, Malaysia. Front. Clim. 4, (2022);
Vienne, A. et al. Enhanced Weathering Using Basalt Rock Powder: Carbon Sequestration, Co-benefits and Risks in a Mesocosm Study With Solanum tuberosum. Front. Clim. 4, (2022).

As enhanced weathering operations move toward greater scale and away from early research and development, there is a need to provide a robust estimate of carbon dioxide removal based on a single approach or reduced sample numbers in the field, wherein the complexity of any actions required in the field should be kept to a minimum, such that the required measurements can be performed and/or necessary samples can be taken and shipped by local personal, e.g. by a farmer working the respective field.

The invention is therefore based on the problem of using only a single or a limited number of measurements in the field while still making sure that the determined measure for the amount of removed carbon dioxide is valid.

The problem is solved by a method for providing a validated measure for the amount of carbon dioxide removed from the atmosphere by a reactant deployed on a field, comprising the steps of
- setting up at least one reference experiment of a first experiment type,
- analyzing a respective field sample taken from at least one field sampling location in the field and/or performing at least one measurement in-situ at the respective field sampling location to determine a field test result that depends on the amount of carbon dioxide removed from the atmosphere by the reactant at the at least one field sampling location,
- analyzing at least one reference sample taken from the respective reference experiment and/or performing at least one measurement in-situ in the respective reference experiment to determine a first reference test result for the first experiment type,
- evaluating a consistency condition, wherein a fulfilment of the consistency condition depends on the field test result and the first reference test result, and
- providing the validated measure for the amount of carbon dioxide removed from the atmosphere by the reactant deployed on the field, when the consistency condition is fulfilled, wherein the validated measure depends on the field test result and/or the first reference test result.

In this document, the term measure for the amount of carbon dioxide removed from the atmosphere by a reactant is used interchangeably with the term CDR-measure (carbon dioxide removal measure), since it measures the amount of carbon dioxide removed from the atmosphere by binding it to the reactant.

The invention is based on the idea, of using at least one reference experiment of a first experiment type that can be set up in controlled conditions and/or that facilitates the taking of samples and/or the performance of in-situ measurements compared to the normal conditions in the field, to validate or even enhance the field test result. In particular reference experiments of multiple experiment types can be used. This will be discussed in more detail later.

As discussed in more detail below, reference experiments are preferably set up in such a way, that the weathering process and therefore the removal of carbon dioxide from the atmosphere is expected to happen at the same rate or at least at a similar rate as in the field and that preferably the chemistry in the various involved phases, e.g. in a solid phase, especially in the soil, in a fluid phase, especially in soil water, in an exchange gas between the soil and the water and/or in the vegetation is comparable between the field and the respective reference experiment.

In this case it is, e.g., possible, that the field test result is only based on a single determined parameter for the respective field sampling location, e.g., on the amount of the remaining rock powder that can, e.g., be determined based on cation concentrations in the soil. Alternatively, the field test result can be based on a low number of parameters.

The first reference test result can then, e.g., describe or be based on the value or values for the same parameter or parameters as the field test result and additionally on values for additional parameters, e.g., a cation concentration for at least one cation and an anion concentration for at least one anion in the soil water. Since a larger number of relevant parameters are known for the reference experiment, a CDR-measure can be determined with a higher accuracy and robustness for the reference experiment. The reference experiment can especially be used to determine values for parameters that would be challenging, expensive and/or work intensive to determine in the field.

In a preferred embodiment, the consistency condition is based on a statistical analysis of a parameter or multiple parameters given for multiple field sampling locations by the field test result and/or of at least one derived parameter that is determined based on the respective parameter or parameters for the respective field sampling location. The fulfilment of the consistency condition can, e.g., depend on a consistency check that checks, whether at least one parameter given by or derived from the field test result, e.g., the CDR-measure, is different from a corresponding parameter given by or derived from the first reference test result for the first experiment type with a given statistical significance level.

It is also possible to additionally analyze the consistency of the field test result and/or the first reference test result with a respective further reference test result for at least one further experiment type.

To allow for an easier understanding of the core idea of the invention, a simpler example for a consistency condition, that only tests the consistency between test results for a single field sampling location and reference experiments of a single experiment type will be discussed as an initial example. The consistency check can, e.g., check, whether the CDR-measure determined for the field sample location based on the field test result is sufficiently similar to the CDR-measure determined for the reference experiments based on the first reference test result. It is, e.g., possible that the reference test result describes a statistical distribution of the CDR- measures in these reference experiments or that such a statistical distribution is determined based on the first reference test result. The consistency condition can then, e.g., be fulfilled or only be fulfillable, when the CDR-measure for the field deviates by less than one or two standard deviations from the mean or median CDR-measure for the reference experiments. Additionally or alternatively, the consistency check can check whether parameters, for which values are determined both in the field and in the reference experiments, have sufficiently similar values for the field and for the reference experiments. Values can be sufficiently similar, when the value for the field deviates by less than one or two standard deviations from the value for the reference experiments.

When the consistency condition is fulfilled, the measure for the amount of carbon dioxide removed from the atmosphere by the reactant can be based exclusively on the field test result. The first reference test result is only used to provide the validation in this case. This can be advantageous to avoid errors due to the differences between the conditions in the field and in the reference experiment.

When a sufficiently close match between the field conditions and the conditions of the reference experiment can be assumed, it can however be advantageous, to calculate the CDR-measure from the values provided by the first reference test result or to take these values into account, e.g., by using a weighted sum of the CDR-measures that are determined based on the field test result and based on the first reference test result.

Various algorithms for determining a CDR-measure based on the values of one parameter or more parameters are known. Approaches for estimating the amount of bond CO₂ are, e.g., known from the initially cited publications and their supplements as well as from the further documents cited therein and will therefore not be discussed in detail. In general, the determination of the respective CDR-measure based on the value for a single parameter or on the values of multiple parameters can be based on a heuristic algorithm that is parametrized or trained based on prior experiments and/or data taken from literature. Such an algorithm can, e.g., be implemented by fitting a model to experimental data, by training an algorithm by machine learning based on this data or even by a simple look-up-table based on a statistical analysis of experimental data.

The reference experiment and all further reference experiments discussed below are preferably set up to have essentially the same soil composition as the field or at least a sufficiently similar soil composition. In particular a sufficiently similar soil composition can be achieved by using open soil close to the field, e.g., in a distance of less than 100 m or less than 1 km or less than 10 km to a border of the field, to set up the reference experiment or the respective further reference experiment. Alternatively, a sufficiently similar soil composition can, e.g., be achieved by taking the soil used for the reference experiment or the respective further reference experiment from the field or from open soil that is sufficiently close to the field, e.g., in a distance of less than 100 m or less than 1 km or less than 10 km or less than 100 km to a border of the field.

Additionally, the amount of reactant deployed per surface area of the reference experiment is preferably approximately identical, e.g. having a difference of less than 20% or less than 10% or less than 5%, to the amount of reactant deployed per surface area of the field. The same reactant should be used in the field and in all reference experiments.

Preferably the same type or types of plants and in particular also at least approximately the same density of plants is grown in the field and in the reference experiment and/or the respective further reference experiment during a time interval between the deployment of the reactant and the determination of the respective test result. It should however be noted that certain types of plants cannot be grown in certain types of reference experiments. It is therefore possible that the given conditions only apply to some of the reference experiments or that they do not apply at all.

As discussed below, the setup of the reference experiment can differ from the conditions in the field, e.g., by adding additional measuring and/or sampling infrastructure in the area of the reference experiment, by enclosing the reference experiment within a closed structure, e.g., greenhouse or laboratory, by separating the soil of the reference experiment from the soil of the field by at least one artificial barrier, etc.

At least one respective further reference experiment can be set up for at least one further experiment type. In this case at least one further reference sample can be taken from the respective further reference experiment and/or at least one further measurement can be performed in-situ in the respective further reference experiment to determine a respective further reference test result for the respective further experiment type, wherein the fulfilment of the consistency condition additionally depends on the respective further reference test result.

The consistency condition can especially comprise multiple partial consistency conditions. In particular the consistency condition can only be fulfilled, when all of the partial consistency conditions are fulfilled.

In an advantageous embodiment, the further experiment type or types can be performed in conditions that are further removed from the conditions in the field sampling locations than the conditions of the first experiment type. As discussed in more detail below, the first reference experiment can, e.g., be performed in relatively natural conditions, while the respective further reference experiment can, e.g., be performed in a greenhouse or a laboratory. In particular, the experiment types can form an ordered sequence of experiment types, wherein the first experiment type is closer to the field conditions than any one of the further experiment types and/or wherein each further experiment type except for the last further experiment type in this ordered sequence is closer to the field conditions than the following further experiment type in the ordered sequence.

The fulfilment of a first one of the partial consistency conditions can depend on the field test result and the first reference test result for the first experiment type and can in particular perform the consistency check, that was already discussed above, for these results. A second partial consistency condition can then depend on the first reference test result and on the further reference test result for the first further experiment type and can in particular perform the consistency check discussed above, wherein the first reference test result is used instead the respective field test result and the further reference test result for the first further experiment type is used instead of the first reference test result. A third partial consistency condition can then depend on the further reference test result for the first further experiment type and the further reference test result for a second one of the further experiment types, etc.

With the approach discussed above, it is especially possible to take test results from at least one further experiment type into account, that is operating in conditions that are rather far removed from the natural conditions in the field. The difference between these rather different conditions can however be bridged by operating the reference experiment and optionally one or more further reference experiments in intermediate conditions and by checking a pairwise consistency between neighboring experiments in this chain of different operating conditions.

The respective further experiment type can differ from the at least one field sampling location and/or from the first experiment type and/or from the other used further experiment type or types by the presence or absence of at least one of the following differentiating features:
- a use of an artificial barrier separating the soil used in the respective reference experiment from the soil in the field,
- the use of a controlled environment, in particular the use of purely artificial irrigation, in the respective reference experiment, and/or
- the absence of vegetation planted in the soil of the respective reference experiment.

It was found that with an increasing number of the listed differentiating features to the conditions at the field sampling location, the taking of samples and the performance of in-situ measurements can be made easier.

The previously discussed ordered sequence of experiment types can especially be formed by adding a respective one of the differentiating features in each step of this ordered sequence. It should be noted that it is also possible that multiple ones of the given differentiating features are added in a single step, thereby essentially skipping one or more experiment types, that will not be used. In other words, the order of the experiment types in the ordered sequence can correspond to the number of differentiating features in the respective type to the field conditions.

An artificial barrier is, e.g., formed when the soil of the respective reference experiment is arranged in a pot or an experimental column. Such an artificial barrier is obviously absent in the field sampling locations, but is typically also absent when a so-called field monitoring station (FMS) is used.

A field monitoring station can, e.g., be implemented in a dedicated area of the field in which sampling is performed with a high density and can, e.g., be supported by permanently installed sensors and/or tools. The field monitoring station can especially be equipped to allow for a taking of soil and water samples at multiple depth within the soil. A taking of samples at multiple depth is typically not plausible for field sampling locations. It is, e.g., possible, that the distance between the field sampling locations is larger by a factor of at least 5 or at least 10 or at least 20 than the distance between multiple reference experiments in a field monitoring station. A field of monitoring station can, e.g., comprise sampling locations and therefore separate reference experiments spaced apart by less than 10 m, especially by less than 2 m, while only a single field sampling location can be used in a relatively large area, e.g., in an area of 10 ha.

A single field monitoring station can optionally comprise multiple areas, wherein a different amount of reactant is deposited in each of these areas. This can allow for internal consistency checks between areas with different concentrations of the reactant, since the CDR-value and other determined parameters should at least approximately scale with the amount of the deposited reactant. The use of the field monitoring station therefore provides a robust reference test result, due to the density of the sampling and due to the optional use of internal consistency checks.

A controlled environment, in particular the use of purely artificial irrigation, can allow an at least partial isolation of the reference experiment from the open atmosphere, e.g., from rainfall, an outside temperature and/or humidity. Therefore, such an experiment can closely match the atmospheric conditions in the field, even when the respective reference experiment is performed quite far from the field, e.g., in a distance of at least 50 km or at least 100 km. This can especially be advantageous, when the field sampling locations are quite remote and accessing the field sampling locations is therefore quite laborious for skilled personnel that might not be locally available. This feature can in particular be realized for indoor experiments, e.g., in a greenhouse and/or in a laboratory.

An absence of vegetation is, e.g., typically preferred, when reference experiments are performed in lixiviation columns, that are also called leaching columns. it is also possible, that the actual vegetation in the field cannot be matched in greenhouse or laboratory conditions, therefore requiring the absence of vegetation in these experiments.

The measurements in the field and the different experiment types can be considered to form multiple experimental pillars, in which measurements can be performed. A mathematical model can also be used as an additional pillar for the validation, as discussed in more detail later.

The field test result can comprise or be based on a result of a first type of measurement, wherein on the one hand the first reference test result comprises or is based on a result of at least one second type of measurement that is not used to determine the field test result and/or wherein on the other hand the respective further reference test result comprises or is based on a result of at least one further type of measurement that is not used to determine the field test result and/or that is not used to determine the first reference test result and/or that is not used to determine any other one of the further reference test results.

By performing different types of measurement in different ones of the experimental pillars discussed above, at least some of the measurements can be performed in the actual conditions in the field or at least under relatively similar conditions to the conditions in the field, while some measurements, that can be elaborate and/or expensive to perform locally in the field, can be performed in controlled conditions. This can allow for a robust determination and validation of the CDR-measure with relatively low effort and cost.

The first experiment type and/or the respective further experiment type can be chosen from a list comprising:
- field monitoring station experiments performed in open natural soil and in natural atmospheric conditions,
- mesocosm experiments performed in a soil volume within an artificial barrier and in natural atmospheric conditions,
- pot experiments performed in in a soil volume within an artificial barrier and in a controlled environment, in particular in a greenhouse or laboratory, and/or
- column experiments that use a column of soil enclosed by an artificial barrier in a controlled environment, in particular in a greenhouse or laboratory.

All experiment types, except for the column experiments, can use vegetation, in particular essentially the same type and density of vegetation as used in the field sampling locations. A more detailed discussion of these possible experiment types is, e.g., given in the previously cited paper by Clarkson, M. O. et al.

The different types of experiments or experimental pillars can be considered to form an ordered sequence, in which field monitoring station experiments are closest to the natural conditions at the field sampling locations, the mesocosm experiments are further removed from the natural conditions, the pot experiments are even further removed from the natural conditions and the column experiments are furthest removed from the natural conditions. The measurements and/or sample extractions performed in field sampling locations, the field monitoring station experiments, the mesocosm experiments, the pot experiments and the column experiments can therefore be considered to form and ordered sequence of five experimental pillars, in which the same type and/or different types of measurement can be performed for cross-validation purposes.

The most robust comparison and therefore consistency check is possible between neighboring experimental pillars or experiment types in this ordered sequence. Therefore, it can be advantageous to select a contiguous subgroup of this ordered sequence as the first and further experiment type used for the and the further reference experiments. It can however also be possible, to skip single experimental pillars or experiment types and therefore, e.g., performed only the first and third experiment type in this ordered sequence.

By using all or at least multiple ones of these experimental pillars, a very robust validation reaching from strongly abstracted laboratory experiments to the actual field conditions with intermediate steps to ensure consistency can be ensured. In many use cases it can however be sufficient, to only use a single one or exactly two or three types of reference experiments, e.g., to only establish a robust consistency between field conditions and a laboratory experiment that allows for an easy determination of additional parameters that allow a more robust estimation and supervision of the carbon dioxide binding to the reactant and therefore of the carbon dioxide removal from the atmosphere.

The fulfilment of the consistency condition can, in particular, depend on results of at least two or at least three or all of the following types of measurement:
- at least one solid measurement type concerning the composition of soil in the field sampling location and/or soil used in the reference experiment and/or the respective further reference experiment,
- at least one fluid measurement type concerning the composition of soil water in the field sampling location and/or the reference experiment and/or the respective further reference experiment,
- at least one gas measurement type concerning the composition of an exchange gas between the soil and the atmosphere in the field sampling location and/or the reference experiment and/or the respective further reference experiment, and
- at least one vegetation measurement type concerning the composition of vegetation growing in the soil in the field sampling location and/or the reference experiment and/or the respective further reference experiment.

The composition of the various relevant "phases", namely the solid phase, the fluid phase, the gaseous phase and the vegetation phase, can therefore be analyzed and the presence of elements and compounds that are involved in the enhanced weathering in the various available storage locations and transport pathways can be taken into consideration, to generate a complete picture of all or at least most relevant processes.

The different measurement types or in other word the different "phases" for which measurements are performed can be considered to form different components of the consistency check. Additionally known properties of the reactant, e.g., of a rock powder forming the reactant, can also be taken into account as a fifth component of the consistency check. It is, e.g., possible to take physical properties, like grain size, a mineralogy and/or chemical properties of the reactant into account.

In principle the different measurement types could be repeated for each one of the five experimental pillars discussed above. As previously discussed, it can however be advantageous to only perform a single type of measurement or just a few types of measurement for the field sampling locations or in general in or for samples taken from natural conditions to simplify the data acquisition. Other types of measurement, in particular from the list given above, can then be performed in at least partially controlled conditions or for samples taken from experiments in such conditions, e.g., mesocosm, pot and/or column experiments.

When the types of measurement are distributed between the given experimental pillars, it is especially advantageous to perform at least one solid measurement type in at least one of the experimental pillars, at least one fluid measurement type in at least one of the experimental pillars, at least one gas measurement type in at least one of the experimental pillars, and at least one vegetation measurement type in at least one of the experimental pillars.

The fulfilment of the consistency condition can, in particular, depend on
- a measurement of an amount of reactant within the soil as the solid measurement type, and/or
- a measurement of a concentration of at least one cation and/or an amount of dissolved inorganic carbon and/or a total alkalinity and/or a pH-value and/or an electrical conductivity and/or a concentration of at least one anion as a respective fluid measurement type, and/or
- a measurement of a CO₂ concentration in the exchange gas as the gas measurement type, and/or
- a measurement of a concentration of at least one cation as the vegetation measurement type.

It was found, that the results of the types of measurement given above are especially relevant to robustly validate the CDR-measure. It was found to be advantageous to perform each of the given types of measurement or all but one of the given types of measurement in at least one of the experimental pillars given above. It is, e.g., typically sufficient to measure only one of the dissolved inorganic carbon and the total alkalinity, since the dissolved inorganic carbon can be determined form the pH-value and the total alkalinity and vice versa.

The individual measurements and their general relevance for the CDR-measure are already known from the initially cited prior art. A good overview of the various types of measurement is, e.g., given by the cited review article by Clarkson, M. O. et al. Therefore, the following discussion only provides an overview of particularly relevant aspects of the various measurement techniques, without explicitly reciting all details that can, e.g., be taken from the cited prior art.

By comparing the amount of reactant left in the soil after a certain time with a known initial amount of reactant, a robust upper bond for the CDR-measure can be established by multiplying the difference between the initial and the current amount by a CDR potential of the reactant. This approach however tends to overestimate the CDR-measure, since neither an export of the reactant into the river-ocean system nor a possible dissolution by acids other than carbonic acid is taken into account. The amount of reactant can therefore act as an upper bound during the validation and/or be corrected by using an empirical model that can then be validated by using at least some of the other measurement types in the same and/or at least one different experimental pillar or experiment type.

The amount of reactant can, e.g., be determined based on the concentration of certain cations, e.g., Calcium and/or Magnesium and/or Sodium and/or Potassium cations, that can, e.g., be determined using mass spectrometry on a soil sample. Since the initial distribution of the reactant can be inhomogeneous, the concentrations of immobile trace elements like, e.g., Titanium or Aluminum, can be used to take initial amounts of the reactant in the respective field sampling location or experiment into account. This approach is discussed in detail in the paper by Reershemius, T. et al. that was already cited in the introduction.

In column, pot and mesocosm experiments, soil water can be collected as a leachate that has penetrated the soil. Infield sampling may be done via the collection of pore waters through MacroRhizon syringes, via soil lysimeters, e.g., suction cup or tension lysimeters, or even via shallow wells at specific depths in the soil column.

Well known analysis techniques can then be applied to the respective water sample to determine cation and/or anion concentrations, the amount of dissolved inorganic carbon and/or the total alkalinity and/or the pH-value and/or the electrical conductivity. The electrical conductivity reflects the total ion concentration in the soil water and hence can approximately describe the accumulation of weathering products.

The anion concentration can help to understand the quality of the measurement and the acidity source, e.g., to what degree the acidity is caused by carbonic acid and strong acids respectively. It is, e.g., possible, to determine concentrations for Cl⁻ , SO₄²⁻, NO₃⁻ and/or HCO₃⁻ anions. Determined anion concentrations can also allow for a correction of cation concentrations, via charge balance, assuming that all remaining positive charges after subtracting the equivalent charges of Cl⁻ , SO₄²⁻, NO₃⁻ anions from the cation charge are charge balanced by HCO₃⁻.

The amount of dissolved inorganic carbon in the soil water indicates the actual amount of carbon ions in the soil water. The amount of dissolved inorganic carbon can be directly determined. It is however also possible to validate or determine the amount of dissolved inorganic carbon based on the total alkalinity and the pH-value.

Vegetation harvested from the respective field sample site and/or reference experiment can be analyzed, e.g., by mass spectrometry. By analyzing the cation concentration in the vegetation, an absorption of reaction products by the vegetation can be taken into account.

The gas measurement can, e.g., be performed by temporarily enclosing a gas volume above the soil surface, e.g. by lowering an otherwise enclosed vessel with an open bottom onto the surface, and monitoring the change of a gas composition, especially of the CO₂ content of the gas, in this volume over time. Other gas fluxes, e.g., CH₄ and N₂O fluxes, which can also be influenced by rock powder application, can also be monitored. The gas measurement can especially be used to take a contribution of microbes to the absorption and release of CO₂ into account and can, e.g., serve as a prove that microbes do not lead to an increase of the overall CO₂ output when the reactant is added to the soil.

Only a single type of measurement can be performed in the at least one field sampling location and that the same type of measurement can also be performed in the field monitoring station experiments. Additionally or alternatively, only a single type of measurement can be performed in the field monitoring station experiments and the same type of measurement can be performed in the at least one field sampling location and/or in the mesocosm experiments. Additionally or alternatively only a single type of measurement can be performed in the mesocosm experiments and the same type of measurement can be performed in the field monitoring station experiments and/or in the pot experiments. Additionally or alternatively, only a single type of measurement can be performed in the pot experiments and the same type of measurement can be performed in the mesocosm experiments and/or in the column experiments. Additionally or alternatively, only a single type of measurement can be performed in the column experiments and the same type of measurement can be performed in the pot experiments.

When the measurements performed in field sampling location, the field monitoring station experiments, the mesocosm experiments, the pot experiments and the column experiments are considered to form an ordered sequence of experimental pillars, the discussed conditions ensure, that the same measurement type is repeated in at least one of the adjacent experimental pillars, when only a single type of measurement is used in an experimental pillar. This can ensure a robust inter-pillar validation, even when only a single type of measurement is used in an experimental pillar. This approach can especially allow for the use of a single type of measurement in a single experimental pillar, especially for the at least one field sampling location, to determine the CDR-measure, while the other experimental pillar or experimental pillars ensure a robust validation.

When a first measurement regime comprises only a single type of measurement of the solid measurement type and a second measurement regime comprises at least one fluid measurement type, or when the first measurement regime comprises only a single type of measurement of the fluid measurement type and the second measurement regime comprises at least one solid measurement type, preferably at least one of the following conditions applies:
- the first measurement regime is used for the at least one field sampling location while the second measurement regime is used for the field monitoring station experiments and/or the mesocosm experiments,
- the first measurement regime is used for the field monitoring station experiments while the second measurement regime is used for the at least one field sampling location and/or for the mesocosm experiments and/or for the pot experiments,
- the first measurement regime is used for the mesocosm experiments while the second measurement regime is used for the at least one field sampling location and/or for the field monitoring station experiments and/or for the pot experiments and/or for the column experiments,
- the first measurement regime is used for the pot experiments wherein the second measurement regime is used for the field monitoring station experiments and/or for the mesocosm experiments and/or for the column experiments,
- the first measurement regime is used for the column experiments wherein the second measurement regime is used for the mesocosm experiments and/or for the pot experiments.

When the previously discussed ordered sequence of five experimental pillars is considered, applying at least one of the discussed conditions will result in the use of a fluid measurement type within two experimental pillars from an experimental pillar, in which a single solid measurement type is used as the only type of measurement and/or the use of solid measurement type within two experimental pillars from an experimental pillar, in which a single fluid measurement type is used as the only type of measurement. It was found, that in cases in which a single type of measurement is used in a single experimental pillar, especially for the at least one field sampling location, to determine the CDR-measure, the cited additional condition can noticeably increase the robustness of the validation.

When a group of experiment types is considered, that either comprises a field experiment type corresponding to the determination of the field test result and the first experiment type or that comprises the field experiment type and the first experiment type and the at least one further experiment type,
- a first selected experiment type selected from the group of experiment types can comprise a measurement of a concentration of at least one cation in the soil water of the first selected experiment type, and
- a second selected experiment type selected from the group of experiment types, that is different from the first experiment type, can comprise a respective measurement of firstly a pH-value and the concentration of at least one anion and an electrical conductivity and secondly an amount of dissolved inorganic carbon and/or a total alkalinity in the soil water of the second selected experiment type.

The robustness of the validation can noticeably increase, when the discussed properties of the soil water are sampled across different experiment types. It should be noted, that each of the experiment types in the group of experiment types corresponds to one of the experimental pillars discussed above. It was found that it can be sufficient, to just sample the cation concentration in one of the experiment types, while the other measurements discussed above are performed for reference experiments of a different experiment type. It should be noted, that the measurement of the cation concentration can optionally be repeated for the second selected experiment type to further improve the robustness of the validation.

In a preferred embodiment, none or only a subset of the types of measurement that are performed in the second selected experiment type are performed in the first selected experiment type. The first selected experiment type can in particular be closer to natural conditions and therefore to the field experiment type than the second one of the selected experiment types. In particular, the second selected experiment type can be performed in a controlled environment, in particular in a greenhouse or laboratory.

The field test result and/or the first reference test result and/or the further or at least one of the further reference test results can comprises or be based on a concentration of at least one anion in soil water collected from open natural soil in natural atmospheric conditions.

The at least one anion concentration can therefore, e.g., be determined for the field sampling locations and/or for the field monitoring station experiments. The measurement of at least one anion concentration is especially relevant for determining the types of acid present in the soil water and can therefore, e.g., allow for an estimate of the influence of acids other than carbonic acid. Since the presence of other acids can be strongly influenced by the composition of rainwater and/or soil chemistry, the at least one anion concentration should preferably be monitored in natural conditions.

The field test result can comprise multiple partial field test results, wherein each partial field test results concerns a respective one of the field sampling locations, wherein the first reference test result comprises multiple partial first reference test results, wherein each partial first reference test result concerns a respective one of the reference experiments, wherein the fulfilment of the consistency condition depends on a statistical analysis of the partial field test results and the partial first reference test results.

It was found, that, in particular for the field of sampling locations and any experiments performed in natural atmospheric conditions, a multitude of different factors can lead to a significant variation in measurement results determined for different field sampling locations and between measurement results determined for different reference experiments and/or further reference experiments of the same experiment type. Therefore, a direct comparison between a single reference experiment and a single field sampling location can be error prone and it can be advantageous to instead or at least additionally compare statistical distributions of the measurements performed in the respective conditions and/or of parameters that are determined from these measurements.

In particular the fulfilment of the consistency condition can depend on whether there is a statistically significant difference between at least one parameter described by or determined from the partial field test results and the same parameter described by or determined from the partial first reference test results at a given statistical significance level.

It is, e.g., possible that the respective partial field test result describes a respective CDR-measure determined for the respective field sampling location based on the measurement or measurements performed for the respective field sampling location and that the respective partial first reference test result describes a respective CDR-measure determined for the respective reference experiment based on the measurement or measurements performed for the respective reference experiment. In this case, the consistency condition can, e.g., only be fulfilled, when there is no statistically significant difference between the CDR-measures for the field sampling locations and for the reference experiments at a given statistical significance level.

Additionally or alternatively, the fulfillment of the consistency condition can depend on a statistical analysis of any other parameter that can be determined from the respective measurements and/or on a direct statistical analysis of at least some of the respective measurements. If the same type of measurement is used in both the field of sampling locations and the reference experiments, it is, e.g., possible to directly determine, whether there is a statistically significant difference between the measurements on the one hand in the field sampling locations and on the other hand in the reference experiments. The consistency condition can, e.g., only be fulfilled, if no such statistically significant difference is detected.

A statistical analysis can also be performed for the further reference experiments of the respective further experiment type. The respective further reference test result for the respective further experiment type can comprise a plurality of partial further reference test results concerning a respective one of the further reference experiments for that further experiment type. The consistency condition can then, e.g., evaluate, whether there is a statistically significant difference between the results, e.g., between determined CDR-measures, between different experiment types at a given statistical significance level.

Additionally or alternatively, the consistency check can, e.g., be based on a theoretical model of the enhanced weathering process, that allows the determination of theoretical values for the measurement results given by the field test result and for the measurement results given by the reference test results and/or of theoretical values for the CDR-measure determined for both of these cases. In this case, the consistency condition can, e.g., be fulfilled, when the value or values given by the field test result and/or the CDR-measure determined based on this value or these values can be matched sufficiently closely, e.g. with an error of less than 10% or less than 20%, by a first parametrization of the theoretical model, the value or values given by the reference test result and/or the CDR-measure determined based on this value or values can be matched sufficiently closely, e.g. with an error of less than 10% or less than 20%, by a second parametrization of the theoretical model, and when the second parametrization is sufficiently close, e.g., within a given tolerance corridor, to the first parametrization. The tolerance corridor for the parametrization can take inaccuracies in the matching of the reference experiment to the field conditions and/or variables that are unknown or not known with sufficient precision, e.g., minor variations in the local climate, fertilizer use and/or the local density of the reactant, into account.

To further improve the robustness of the validation of the measure for the amount of removed carbon dioxide, and/or to allow for a similar robustness of the validation while lowering the necessary number of reference experiments or reference experiment types, a mathematical model can be used as part of the validation process.

It is therefore possible, that a mathematical model that models at least one process that is part of the sequestration of carbon dioxide from the atmosphere using the reactant is used to determine at least one model result, wherein the respective model result corresponds to an expected value of at least one measurement result, wherein the respective measurement result forms or is part of the field test result or the first reference test result or the further reference test result or one of the further reference test results, wherein the fulfilment of the consistency condition additionally depends on the respective model result.

In other words, the consistency of the field test result and/or the first reference test result and/or the further reference test result or at least one of the reference test results with the mathematical model can be checked as part of the consistency condition. A comparison of measured parameters with models in the context of carbon dioxide removal from the atmosphere is, e.g., already discussed in the initially cited paper by Vienne, A. et al. for the concentration of Magnesium and Calcium. A more elaborate model that can be used to validate a plurality of measured parameters is, e.g., disclosed in Bertagni, M. B. et al. Advancing Enhanced Weathering Modeling in Soils: Systematic Comparison and Validation with Experimental Data. ESS Open Archive . January 18, 2024 doi: 10.22541/essoar.170559500.09183720/v1

A combination of a validation via the at least one reference experiment type, as discussed above, and a validation via a mathematical model can be especially robust. It is, e.g., possible to use the mathematical model to validate the reference experiments for at least one of the reference experiment types, since such a reference experiment can be easier to model than the carbon dioxide removal in the field.

Further details and features of the invention will now be discussed with reference to exemplary embodiments of the invention and the accompanying figures. The figures are schematic representations showing:
- Fig. 1: an ordered sequence of experimental pillars that can be used to implement the method according to the present invention,
- Fig. 2: a flowchart of an exemplary embodiment of the method according to the present invention, and
- Fig. 3: a chart showing distributions of different types of measurement across different experimental pillars for two different embodiments of the method according to the present invention.

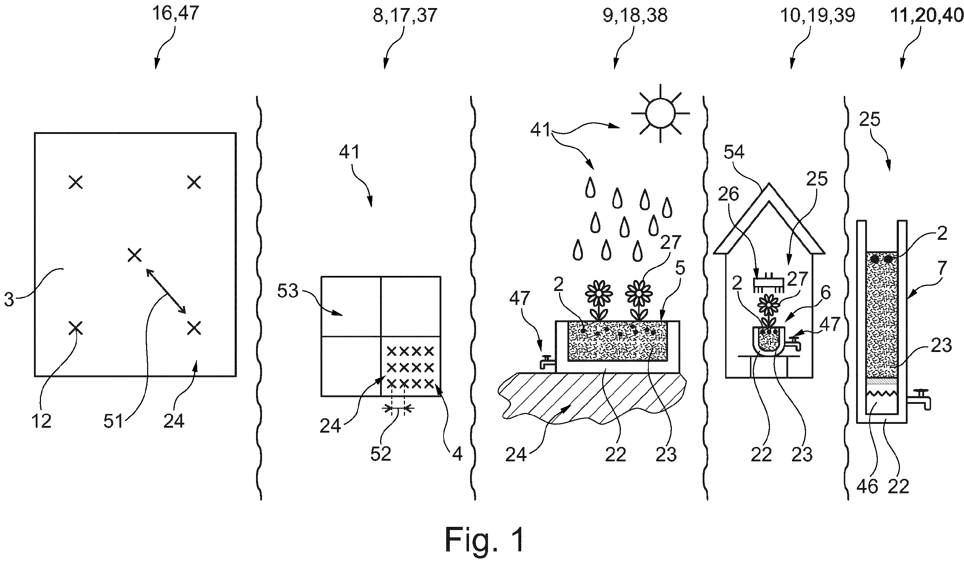

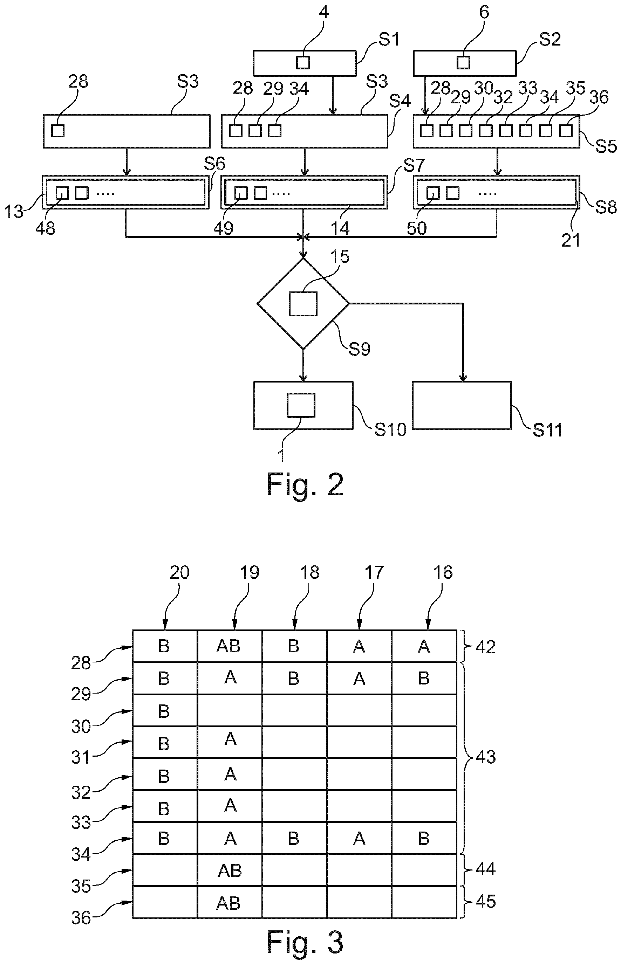

Fig. 1 shows a schematic representation of various experimental pillars that can be used to implement a method for providing a validated measure 1 for the amount of carbon dioxide removed from the atmosphere by a reactant 2 deployed on a field 3. Due to the large scale for of the depictions of for the pillars 16 and 17, the reactant 2 itself is only shown for the pillars 18 to 20. A flowchart of an exemplary embodiment of the method will later be discussed in detail with reference to fig. 2.

The core idea of the discussed method is to gather information from multiple ones of the experimental pillars 16-20 shown in fig. 1, e.g. from two, tree, four or all of these pillars 16-20, and to evaluate a consistency condition 15, to check, whether the results from the different considered pillars 16 to 20 are consistent. If this is the case, a measure for the amount of carbon dioxide removed from the atmosphere by the reactant deployed on the field that is determined based on the gathered information, especially based on information that is exclusively gathered from the pillar 16 and therefore from measurements and samples are taken from the actual field of 3, is considered to be a validated measure 1 for the amount of removed carbon dioxide. In the cases of an inconsistency between experimental pillars 16-20, an error handling can be initiated, that, e.g., determines the measure for the removed carbon dioxide based on a more conservative model or that reduces an initially determined measure by a given factor or offset to ensure that the inconsistencies do not lead to an overestimation of the CDR-value.

The validation of the measure of removed carbon dioxide is especially relevant, when, e.g., the owner of the field is reimbursed for the amount of removed carbon dioxide, e.g., when the removal of carbon dioxide from the atmosphere is used to offset or reduce the carbon dioxide footprint of a company. In this case it is, e.g., possible to only take validated measures 1 for the amount of removed carbon dioxide into account or to reduce their reimbursement when it was found that the measure cannot be validated.

As discussed later with reference to the flowchart in fig. 2 and to the overview over the types of measurement 28-36 used in the various pillars 16-20 in two different embodiments of such a method, it can be sufficient, to use information gathered from a subset of the pillars 16 to 28 shown in fig. 1 to perform a robust validation.

While the first experimental pillar 16 concerns samples and measurements that are taken from the actual field 3, the further experimental pillars 17 to 20 each correspond to respective reference experiments 4-7 of a respective experiment type 8 to 11, as discussed below.

The first experimental pillar 16 is a field experiment type 47 in which samples and/or measurements taken in-situ are collected for multiple field sampling locations 12 in the field 3. This pillar 16 is used in all discussed examples, to determine a field test result 13 that depends on the amount of carbon dioxide removed from the atmosphere by the reactant 2 at the field sampling locations 12. the field sampling locations 12 can be spaced rather far apart. It is, e.g., possible, to only use a single field sampling location 12 in each hectare of land. The distance 51 between field sampling locations 12 can therefore, e.g., be more than 50 m or more than 100 m.

In the second experimental pillar 17, field monitoring station experiments 37 are used as the experiment type 8 of the reference experiments 4. The reference experiments 4 are therefore performed in open natural soil 24 and in natural atmospheric conditions 41.

Field monitoring stations are specific areas of open soil that are typically located close to the field 3, such that very similar soil and atmospheric conditions can be expected. The distance 52 between the different reference experiments 4 that are field monitoring station experiments 37 is a lot smaller than the distance 51 between the different field sampling locations 12, e.g., between 2 and 5 meters, therefore allowing for a fast and easy collection of measurements and samples. The field monitoring station can also comprise dedicated equipment for taking samples and/or for measurement acquisition, e.g., permanently installed lysimeters, sensors, etc., further reducing the cost and effort for the sample and measurement acquisition.

In the example in the field monitoring station comprises 3 separate microplots 53, that can, e.g., each have an area of 15 m by 15 m. Different concentrations of the reactant can be used in the different microplots 53. While for simplicity's sake the locations of the reference experiments 4 are only shown for one of the microplots 53, reference experiments 4 can be performed in all of the microplots 53, therefore, e.g., allowing to check a consistency between the measured data and the variation of the reactant concentration between the different microplots 53.

In the third experimental pillar 18, mesocosm experiments 38 are used as the experiment type 9 of the reference experiments 5. The reference experiments 5 are therefore performed in a soil 23 volume that is separated from the soil 24 in the field 3 by an artificial barrier 22. The soil can, e.g., be arranged in a container that is arranged on top of the soil surface, wherein the soil 23 within the container is open to the natural atmospheric conditions 41. Sample taking and/or measurements can be assisted by specific tools, e.g., by providing faucet 47 for a sampling of the soil of water 22.

In the fourth experimental pillar 19, pot experiments 39 are used as the experiment type 10 of the reference experiments 6. The reference experiments 6 are therefore performed in a soil 23 volume within an artificial barrier 22 and in a controlled environment 25, that is provided by a greenhouse 54 in the example. An artificial irrigation 26 is used to essentially replicate in the rainfall pattern in the area of the field 3. Overall, the controlled environment 25 can be controlled in such a way, that the conditions in the field 3 are closely replicated, even when the reference experiments 6 of this experimental pillar 19 are performed far away from the field 3. This is especially useful, when skilled personal that might not be available locally in the area close to the field 3 is necessary to perform at least some of the types of measurement 28 to 36 performed in this pillar 19.

In the fifth experimental pillar 20, column experiments 40 are used as the experiment type 11 of the reference experiments 7. The reference experiments 7 therefore use a column of soil 23 enclosed by an artificial barrier 22 in a controlled environment 25. While the same type and density of vegetation 27 is used in all other experimental pillars 16-10, the column experiments 40 does not use any vegetation. The respective reference experiment 7 can, e.g., be implemented by using a leaching column, therefore allowing for a fast set up and experimental turnaround.

Fig. 2 is a flow chart of an exemplary method for providing a validated measure 1 for the amount of carbon dioxide removed from the atmosphere by a reactant 2 deployed on a field 3, that uses information collected from a multiple ones of the experimental pillars 16 to 20 discussed above. This embodiment is discussed with additional reference to figure 3, that provides an overview, which types of measurement 28-36 are used in which experimental pillar 16 to 20. It should be noted, that the types of measurement 28-36 that are actually used in the flowchart in fig. 2 are marked by the letter "A" in this chart.

In step S1, multiple reference experiments 4, in the example the field monitoring station experiments 37 of the experimental pillar 17, are set up by preparing the field monitoring station.

In step S2, in the example further reference experiments 6, namely the pot experiments 39 of the pillar 19, are set up by setting up the enclosed soil 23 volumes in the greenhouse.

It should be noted, that in different embodiments of the method different ones of the experimental pillars 17 to 20 and therefore experiment types 8-11 could be used to set up the respective reference experiments 4-7 and further reference experiments 4-7. It would also be possible to only use only one of the experimental pillars 17 to 20 in addition to the experimental pillar 16 and to therefore skip steps S2, S5 and S8 or to use multiple different further experiment types 8-11 and therefore to replicate the steps S2, S5 and S8 for multiple experimental pillars 17-20.

In the steps S3 to S5 various types of measurement 28 to 36 can be performed for each one of the considered experimental pillars 16 to 20. Since the various types of measurement 28 to 36 were already discussed in detail in the general part of the description, only a short overview of the possible types of measurement 28 to 36 will be given:
The type of measurement 28 determines an amount of reactant 2 within the soil 23, 24 as a solid measurement type 42.

The types of measurement 29-34 are fluid measurement types 43 that determine properties of the soil water 22 in the field sampling location 12 or the respective reference experiment 8-11. Specifically, the type of measurement 29 determines a concentration of at least one cation, the type of measurement 30 determines an amount of dissolved inorganic carbon, the type of measurement 31 determines a total alkalinity, the type of measurement 32 determines a pH-value, the type of measurement 33 determines an electrical conductivity and the type of measurement 34 determines a concentration of at least one anion.

The type of measurement 35 determines a CO2 concentration in the exchange gas as the gas measurement type 44 and the type of measurement 36 determines a concentration of at least one cation in vegetation 27 harvested from the respective field sampling location 12 or reference experiment 4-6.

As also shown by the letter "A" in the chart in figure 3, in the example only the type of measurement 28 is performed for the experimental pillar 16 and therefore for each one of the field sampling locations 12 in step S3, the types of measurement 28, 29 and 34 are performed for the experimental pillars 17 and therefore for the field monitoring station experiments 37 in step S4, and all of the types of measurement 28 to 36, except for the type of measurement 30 are performed for the experimental pillar 19 and therefore for the pot experiments 39 in step S5. The type of measurement 30 can be skipped, since the amount of dissolved inorganic carbon can be determined from the total alkalinity and the pH-value.

The selection, what types of measurement 28-36 are performed for which one of the experimental columns 16 to 20 is based on various design rules, that were already discussed in detail in the general part of the description. Therefore, only a short overview of the effects of the relevant rules will be given below:
Since only a single type of measurement 28 is used in the experimental pillar 16 in step S3, the same type of measurement 28 is repeated in the neighboring experimental pillar 17 in step S4.

Since the experimental pillar 16 only comprises a single solid measurement type 42, at least one fluid measurement type 43 should be used either in the experimental pillar 17 or in the experimental pillar 18. This condition is satisfied by the use of the types of measurement 29 and 34 in the experimental pillar 17 in step S4.

The use of the type of measurement 34 in the experimental pillar 17 also satisfies the condition, that the concentration of at least one anion should be determined for soil water 46 collected from open natural soil 24 in natural atmospheric conditions 41.

Since the concentration of at least one cation in the soil water 46 is determined in step S2 for the experimental pillar 17 by the type of measurement 29, firstly a pH-value and the concentration of at least one anion and an electrical conductivity and secondly an amount of dissolved inorganic carbon and/or a total alkalinity in the soil water should be determined for at least one of the experimental pillars 16, 18 or 19. This condition is satisfied by the use of the types of measurement 29 and 31 to 34 in step S5 in the experimental pillar 19.

It should also be noted, that the types of measurement 28 to 36 used in the various experimental pillars 16 to 20 are selected in such a way, that the each of at least one solid measurement type 42, at least one fluid measurement type 43, at least one gas measurement type 44 and at least one vegetation measurement type 45 is performed during the steps S3 to S5.

In step S6 a field test result 13 is determined, based on the measurements performed in a step S3. In the example, a respective partial field test result 48 is determined for each one of the individual field sampling locations 12, describing the respective CDR-measure determined for the respective field sampling location 12.

In step S7 a first reference test result 14 is determined based on the measurements performed in step S4. In the example, a respective partial first reference test result 49 is determined for each reference experiment 4 in the experimental pillar 17, describing the respective CDR-measure determined for this reference experiment 4.

In step S8 a further reference test result 21 is determined based on the measurements performed in step S5. In the example, a respective partial further reference test result 50 is determined for each one of the further reference experiments 6 in the experimental pillar 19, describing the respective CDR-measure determined for this further reference experiment 6.

In step S9, a consistency condition 15 is evaluated. In the example the fulfilment of the consistency condition 15 depends on the field test result 13, the first reference test result 14, and the further reference test results 21. In the example, the consistency condition comprises two partial consistency conditions, that both need to be fulfilled to fulfill the consistency condition 15. The first partial consistency condition is fulfilled, when there is no statistically significant difference between the partial field test results 48 and the partial first reference test results 49. The second partial consistency condition is fulfilled, when there is no statistically significant difference between the partial first reference test results 49 and the partial further reference test results 50.

When the consistency condition 15 is fulfilled, the measurements concerning the binding of carbon dioxide to the reactant are consistent between the considered experimental pillars 16-20 and the measurements in step S3 concerning the field sampling locations 12 are therefore valid. A validated measure 1 for the amount of carbon dioxide removed from the atmosphere by the reactant 2, e.g., an average CDR-value for the field sampling locations 12, can therefore be provided in a step S10.

If the consistency condition 15 is not fulfilled, an error handling can be performed in a step S11. The error handling can, e.g., comprising the provision of a lower measure for the amount of carbon dioxide removed from the atmosphere by the reactant, that can, e.g., be determined by using a conservative model to provide a lower bound for the carbon capture.

Besides the combinations of different experimental pillars 16 to 20 with the various types of measurement 28-36 that are used in the method according to figure 2 and that are marked in figure 3 by the letter "A", an exemplary alternative combination is also shown in Fig. 3 and marked by the letter "B".

The measurement strategy marked by the letter "B" distributes the various measurement types 28 to 36 across most of the experimental pillars 16 to 20 and only skips the experimental pillar 17 and therefore the field monitoring station experiments 37. It is possible to skip this column, since the use of only a single type of measurement 28 to 36 in the experimental pillar 16 is avoided. Since the experimental pillar 16 only uses fluid measurement types 43, a solid measurement type 42 should be used in the experimental pillar 17 or 18 and is therefore used in the experimental pillar 18.

It can be easily checked, that the measurement of strategy marked by the letter "B" also meets all of the other requirements are discussed above with respect to the measurement strategy marked by the letter "A". The two given measurement strategies are purely examples of the implementation of the design rules discussed above and it is easy to construct further measurement strategies matching these design rules.

In the example, only actual reference experiments are used to validate the measure for the amount of carbon dioxide removed from the atmosphere. As already discussed in more detail in the general part of the description, it is additionally possible to use a full or partial mathematical model of the carbon dioxide removal as part of the validation. In this case the consistency condition 15 discussed above could be modified to additionally evaluate at least one model result provided by such a model.

## Claims

1. Method for providing a validated measure (1) for the amount of carbon dioxide removed from the atmosphere by a reactant (2) deployed on a field (3), comprising the steps of
- setting up at least one reference experiment (4-7) of a first experiment type (8-11),
- analyzing a respective field sample taken from at least one field sampling location (12) in the field (2) and/or performing at least one measurement in-situ at the respective field sampling location (12) to determine a field test result (13) that depends on the amount of carbon dioxide removed from the atmosphere by the reactant (2) at the at least one field sampling location (12),
- analyzing at least one reference sample taken from the respective reference experiment (4-7) and/or performing at least one measurement in-situ in the respective reference experiment (4-7) to determine a first reference test result (14) for the first experiment type (8-11),
- evaluating a consistency condition (15), wherein a fulfilment of the consistency condition (15) depends on the field test result (13) and the first reference test result (14), and
- providing the validated measure (1) for the amount of carbon dioxide removed from the atmosphere by the reactant (2) deployed on the field (3), when the consistency condition (15) is fulfilled, wherein the validated measure (1) depends on the field test result (13) and/or the first reference test result (14).

2. Method according to claim 1, **characterized in that** at least one respective further reference experiment (4-7) is set up for at least one further experiment type (8-11), wherein at least one further reference sample is taken from the respective further reference experiment (4-7) and/or at least one further measurement is performed in-situ in the respective further reference experiment (4-7) to determine a respective further reference test result (21) for the respective further experiment type (4-7), wherein the fulfilment of the consistency condition (15) additionally depends on the respective further reference test result (21).

3. Method according to claim 2, **characterized in that** the respective further experiment type (4-7) differs from the at least one field sampling location (12) and/or from the first experiment type (4-7) and/or from the other used further experiment type (4-7) or types (4-7) by the presence or absence of at least one of the following differentiating features:
- a use of an artificial barrier (22) separating the soil (23) used in the respective reference experiment (4-7) from the soil (24) in the field (3),
- the use of a controlled environment (25), in particular the use of purely artificial irrigation (26), in the respective reference experiment (4-7), and/or
- the absence of vegetation (27) planted in the soil (23, 24) of the respective reference experiment (4-7).

4. Method according to one of the preceding claims, **characterized**
- **in that** the field test result (13) comprises or is based on a result of a first type of measurement (28-36), and
- **in that** on the one hand the first reference test result (14) comprises or is based on a result of at least one second type of measurement (28-36) that is not used to determine the field test result (13) and/or
- **in that** on the other hand the respective further reference test result (21) comprises or is based on a result of at least one further type of measurement (28-36) that is not used to determine the field test result (13) and/or that is not used to determine the first reference test result (14) and/or that is not used to determine any other one of the further reference test results (21).

5. Method according to one of the preceding claims, **characterized in that** the first experiment type (8-11) and/or the respective further experiment type (8-11) is chosen from a list comprising:
- field monitoring station experiments (37) performed in open natural soil (24) and in natural atmospheric conditions (41),
- mesocosm experiments (38) performed in a soil (23) volume within an artificial barrier (22) and in natural atmospheric conditions (41),
- pot experiments (39) performed in in a soil (23) volume within an artificial barrier (22) and in a controlled environment (25), in particular in a greenhouse (54) or laboratory, and/or
- column experiments (40) that use a column of soil (23) enclosed by an artificial barrier (22) in a controlled environment (25), in particular in a greenhouse (54) or laboratory.

6. Method according to one of the preceding claims, **characterized in that** the fulfilment of the consistency condition (15) depends on results of at least two or at least three or all of the following types of measurements (28-36):
- at least one solid measurement type (42) concerning the composition of soil (24) in the field sampling location (12) and/or soil (23, 24) used in the reference experiment (4-7) and/or the respective further reference experiment (4-7),
- at least one fluid measurement type (43) concerning the composition of soil water (46) in the field sampling location (12) and/or the reference experiment (4-7) and/or the respective further reference experiment (4-7),
- at least one gas measurement type (44) concerning the composition of an exchange gas between the soil (23, 24) and the atmosphere in the field sampling location (12) and/or the reference experiment (4-7) and/or the respective further reference experiment (4-7), and
- at least one vegetation measurement type (45) concerning the composition of vegetation (27) growing in the soil (23, 24) in the field sampling location (12) and/or the reference experiment (4-7) and/or the respective further reference experiment (4-7).

7. Method according to claim 6, **characterized in that** the fulfilment of the consistency condition (15) depends on
- a measurement of an amount of reactant (2) within the soil (23, 24) as the solid measurement type (42), and/or
- a measurement of a concentration of at least one cation and/or an amount of dissolved inorganic carbon and/or a total alkalinity and/or a pH-value and/or an electrical conductivity and/or a concentration of at least one anion as a respective fluid measurement type (43), and/or
- a measurement of a CO₂ concentration in the exchange gas as the gas measurement type (44), and/or
- a measurement of a concentration of at least one cation as the vegetation measurement type (45).

8. Method according to claim 5 and claim 6 or 7, **characterized**
- **in that** only a single type of measurement (28-36) is performed in the at least one field sampling location (12) and the same type of measurement (28-36) is also performed in the field monitoring station experiments (37), and/or
- **in that** only a single type of measurement (28-36) is performed in the field monitoring station experiments (37) and the same type of measurement (28-36) is performed in the at least one field sampling location (12) and/or in the mesocosm experiments (38), and/or
- **in that** only a single type of measurement (28-36) is performed in the mesocosm experiments (38) and the same type of measurement (28-36) is performed in the field monitoring station experiments (37) and/or in the pot experiments (39), and/or
- **in that** only a single type of measurement (28-36) is performed in the pot experiments (39) and the same type of measurement (28-36) is performed in the mesocosm experiments (38) and/or in the column experiments (40), and/or
- **in that** only a single type of measurement (28-36) is performed in the column experiments (40) and the same type of measurement is performed in the pot experiments (39).

9. Method according to claim 8 or according to claim 5 and claim 6 or 7, **characterized in that**
- one the one hand, a first measurement regime comprises only a single type of measurement (28-36) of the solid measurement type (42) and a second measurement regime comprises at least one fluid measurement type (43), or
- on the other hand, the first measurement regime comprises only a single type of measurement (28-36) of the fluid measurement type (43) and the second measurement regime comprises at least one solid measurement type (42),
wherein in both cases at least one of the following conditions applies:
- the first measurement regime is used for the at least one field sampling location (12) while the second measurement regime is used for the field monitoring station experiments (37) and/or the mesocosm experiments (38),
- the first measurement regime is used for the field monitoring station experiments (37) while the second measurement regime is used for the at least one field sampling location (12) and/or for the mesocosm experiments (38) and/or for the pot experiments (39),
- the first measurement regime is used for the mesocosm experiments (38) while the second measurement regime is used for the at least one field sampling location (12) and/or for the field monitoring station experiments (37) and/or for the pot experiments (39) and/or for the column experiments (40),
- the first measurement regime is used for the pot experiments (39) wherein the second measurement regime is used for the field monitoring station experiments (37) and/or for the mesocosm experiments (38) and/or for the column experiments (40),
- the first measurement regime is used for the column experiments (40) wherein the second measurement regime is used for the mesocosm experiments (38) and/or for the pot experiments (39).

10. Method according to one of the preceding claims, **characterized in that**
- a group of experiment types
• either comprises a field experiment type (47) corresponding to the determination of the field test result (13) and the first experiment type (8-11)
• or comprises the field experiment type (47) and the first experiment type (8-11) and the at least one further experiment type (8-11),
- wherein a first selected experiment type selected from the group of experiment types comprises a measurement of a concentration of at least one cation in the soil water (46) of the first selected experiment type (28-36), and
- wherein a second selected experiment type selected from the group of experiment types, that is different from the first experiment type, comprises a respective measurement of firstly a pH-value and the concentration of at least one anion and an electrical conductivity and secondly an amount of dissolved inorganic carbon and/or a total alkalinity in the soil water of the second selected experiment type.

11. Method according to one of the preceding claims, **characterized in that** the field test result (13) and/or the first reference test result (14) and/or the further or at least one of the further reference test results (21) comprises or is based on a concentration of at least one anion in soil water (46) collected from open natural soil (24) in natural atmospheric conditions (41).

12. Method according to one of the preceding claims, **characterized in that**
- the field test result (13) comprises multiple partial field test results (48), wherein each partial field test results (48) concerns a respective one of the field sampling locations (12),
- wherein the first reference test result (14) comprises multiple partial first reference test results (49), wherein each partial first reference test result (50) concerns a respective one of the reference experiments (4-7),
- wherein the fulfilment of the consistency condition (15) depends on a statistical analysis of the partial field test results (48) and the partial first reference test results (49).

13. Method according to one of the preceding claims, **characterized in that** a mathematical model that models at least one process that is part of the sequestration of carbon dioxide from the atmosphere using the reactant (2) is used to determine at least one model result, wherein the respective model result corresponds to an expected value of at least one measurement result, wherein the respective measurement result forms or is part of the field test result (13) or the first reference test result (14) or the further reference test result (21) or one of the further reference test results (21), wherein the fulfilment of the consistency condition (15) additionally depends on the respective model result.
